# EUROPÄISCHE PATENTSCHRIFT

(11) **EP 2 257 514 B1**
(45) Veröffentlichungstag und Bekanntmachung des Hinweises auf die Patenterteilung: **28.10.2015**
(21) Anmeldenummer: 09715510.5
(22) Anmeldetag: 27.02.2009
(51) Int. Cl.: C07C 45/38, C07C 47/21, C07C 45/39

(54) **VERFAHREN ZUR HERSTELLUNG VON OLEFINISCH UNGESÄTTIGTEN CARBONYLVERBINDUNGEN DURCH OXIDATIVE DEHYDRIERUNG VON ALKOHOLEN**
METHOD FOR PRODUCING OLEFINICALLY SATURATED CARBONYL COMPOUNDS BY OXIDATIVE DEHYDROGENATION OF ALCOHOLS
PROCÉDÉ DE PRODUCTION DE COMPOSÉS CARBONYLÉS OLÉFINIQUEMENT INSATURÉS PAR DÉSHYDROGÉNATION OXYDATIVE D'ALCOOLS

(30) Priorität: 28.02.2008 DE 102008011767
(43) Veröffentlichungstag der Anmeldung: 08.12.2010
(73) Patentinhaber: BASF SE, 67056 Ludwigshafen (DE)
(72) Erfinder: LIMBACH, Michael, 67551 Worms (DE); TELES, Joaquim Henrique, 67166 Otterstadt (DE); ABDALLAH, Radwan, 67063 Ludwigshafen (DE); MÄURER, Torsten, 67245 Lambsheim (DE); JOHANN, Thorsten, 67117 Limburgerhof (DE)
(86) Internationale Anmeldenummer: PCT/EP2009/052383
(87) Internationale Veröffentlichungsnummer: WO 2009/106621

(56) Entgegenhaltungen:
- EP-A1- 0 112 261
- EP-A1- 0 881 206
- ALBERTO ABAD, CARLES ALMELA, AVELINO CORMA AND HERMENEGILDO GARCÍA: "Unique gold chemoselectivity for the aerobic oxidation of allylic alcohols" CHEMICAL COMMUNICATIONS - CHEMCOM., 2006, Seiten 3178-3180, XP002536255 GBROYAL SOCIETY OF CHEMISTRY. ISSN: 1359-7345 DOI: 10.1039/b606257a
- ALBERTO ABAD, DR., AVELINO CORMA, PROF. DR., HERMENEGILDO GARCÍA, PROF. DR.: "Catalyst Parameters Determining Activity and Selectivity of Supported Gold Nanoparticles for the Aerobic Oxidation of Alcohols: The Molecular Reaction Mechanism" CHEMISTRY - A EUROPEAN JOURNAL., Bd. 14, Nr. 1, 23. November 2007 (2007-11-23), Seiten 212-222, XP002536256 Wiley - V C H Verlag GmbH & Co. KGaA ISSN: 0947-6539

## Beschreibung

Die vorliegende Erfindung betrifft ein Verfahren zur Herstellung von olefinisch ungesättigten Carbonylverbindungen durch oxidative Dehydrierung von Alkoholen in sauerstoffhaltiger Atmosphäre an einem geträgerten Katalysator.

Die oxidative Dehydrierung von ungesättigten Alkoholen zu Aldehyden ist an sich bekannt und in der Literatur beschrieben.

In der DE-A-25 17 859 wird die Dehydrierung ungesättigter Alkohole an Kupfer-Katalysatoren beschrieben, die im Wesentlichen in Abwesenheit von Sauerstoff durchgeführt wird. Dabei entstehen Gemische verschiedener Aldehyde, die in aufwändigen Trennoperationen in ihre Komponenten zerlegt werden müssen, wenn reine Produkte gewünscht sind.

In der DE-B 20 20 865 und der DE-B-20 41 976 wird die Dehydrierung von β,γ-ungesättigten Alkoholen bzw. α,β-ungesättigten Alkoholen zu α,β-ungesättigten Aldehyden bei Temperaturen im Bereich von 150 bis 600°C beschrieben. Als Dehydrierungskatalysatoren werden auch Mischkatalysatoren, z. B. solche aus Kupfer und Silber genannt. Nachteilig ist dabei, dass man erhebliche Mengen an nucleophilen Substanzen zusetzen muss.

In der US-A 4,154,762 wird ein Verfahren zur Herstellung von Aldehyden und Ketonen beschrieben, nach welchem die entsprechenden Alkohole bei Temperaturen im Bereich von 250 bis 600°C in Anwesenheit eines Gold-Katalysators umgesetzt werden. Dabei wird ein massiver und daher entsprechend teurer Gold-Katalysator verwendet.

In der EP-A-244 632 wird ein Rohrbündelreaktor zur Durchführung katalytischer organischer Reaktionen in der Gasphase beschrieben, der durch bestimmte Dimensionierung der Reaktionsrohrlänge zum Innendurchmesser gekennzeichnet ist. Die oxidative Dehydrierung wird dabei bei Temperaturen im Bereich von 300 bis 600°C an einem geträgerten Katalysator durchgeführt. In der EP-A-881 206 wird ein Verfahren zur kontinuierlichen technischen Herstellung ungesättigter aliphatischer Aldehyde in einem Rohrbündelreaktor beschrieben, nach welchem vor der eigentlichen Reaktion die Reaktionsmischung oberhalb des Taupunktes des eingesetzten Alkohols aber unterhalb der Starttemperatur der Reaktion durch den geträgerten Katalysator geleitet wird. Die eigentliche oxidative Dehydrierung findet auch nach dem in dieser Schrift beschriebenen Verfahren bei Temperaturen von 300°C oder mehr statt.

Aus Angew. Chem. Int. Ed. 2007, 47, Seiten 138-141, ist die oxidationsmittelfreie Alkoholdehydrierung unter Verwendung eines recyclierbaren geträgerten Silberkatalysators auf einem Hydrotalcit-Träger beschrieben. Die Reaktion wird in Abwesenheit von Sauerstoff beispielsweise in Argonatmosphäre durchgeführt. Ausweislich Beispiel 16 in Tab. 1 wird ein olefinisch ungesättigter Alkohol mit einem Phenylsubstituenten zum entsprechenden Aldehyd in Abwesenheit von Sauerstoff umgesetzt.

In Chem. Commun. 2006, 3178-3180 wird die aerobe Oxidation von allylischen Alkoholen unter Verwendung eines geträgerten Goldkatalysators auf einem Ceroxid-Träger beschrieben.

Nach der vorliegenden Erfindung wird ein Verfahren zur Herstellung von olefinisch ungesättigten Carbonylverbindungen der allgemeinen Formel I zur Verfügung gestellt 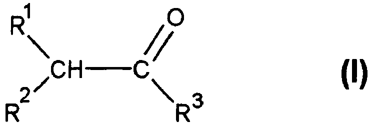 wobei R¹ Wasserstoff ist wenn R² einen Rest der allgemeinen Formel II 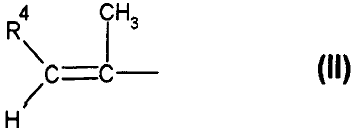 darstellt, oder R¹ und R² zusammen einen Rest der Formel III 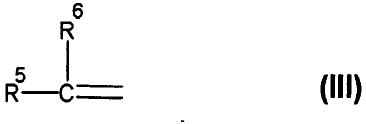 bedeuten, und R³, R⁴, R⁵ und R⁶ jeweils unabhängig voneinander für Wasserstoff, eine C₁ - C₁₈ Alkyl-, eine ggf. substituierte C₅ - C₁₈-Cycloalkyl-, oder eine ggf. substituierte C₆ - C₁₈-Arylgruppe stehen können, wobei die Ringatome der Cycloalkyl- und Arylgruppen teilweise durch Heteroatome, ausgewählt aus N, O und S substituiert sein können, durch oxidative Dehydrierung von Alkoholen der allgemeinen Formel IV in sauerstoffhaltiger Atmosphäre 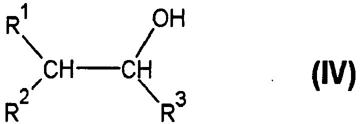 wobei R¹ und R² die vorstehend genannten Bedeutungen haben können, an einem goldhaltigen Katalysator, wobei der goldhaltige Katalysator geträgert ist, Gold oder Mischungen aus Gold und Edelmetallen ausgewählt aus Cu, Ag, Pd, Pt, Rh, Ru, W oder Os enthält und die Reaktion in einem Temperaturbereich von 50 bis 240°C in Anwesenheit von Luft, Wasserstoffperoxid oder einem Gas- oder Gasgemisch mit einem Sauerstoffgehalt von 7-30 Vol% als sauerstoffhaltiger Atmosphäre durchgeführt wird, wobei
als Trägermaterial Aluminiumoxid, ein Alumosilikat oder ein Hydrotalcit oder deren Mischungen eingesetzt wird.

Bevorzugte Ausführungsformen des erfindungsgemäßen Verfahrens sind der nachfolgenden Beschreibung und den Unteransprüchen zu entnehmen.

Nach dem erfindungsgemäßen Verfahren können olefinisch ungesättigte Carbonylverbindungen der allgemeinen Formel I hergestellt werden, wobei in dieser Formel R¹ für Wasserstoff und R² für einen Rest der Formel II stehen oder R¹ und R² zusammen einen Rest der allgemeinen Formel III bedeuten.

Die Substituenten R³, R⁴, R⁵ und R⁶ in den allgemeinen Formel I bis III stehen dabei unabhängig voneinander jeweils für Wasserstoff, eine C₁ - C₁₈ Alkyl-, eine ggf. substituierte C₅ - C₁₈ Cycloalkyl- oder eine ggf. substituierte C₆ - C₁₈ Arylgruppe. Als Substituenten an den Cycloalkyl- oder Arylgruppen kommen vorzugsweise C₁ - C₆ Alkylreste oder C₁ - C₆ Alkoxyreste in Frage. Beispielhaft seien für Alkohole der allgemeinen Formel IV 3-Buten-1-ol, 3-Penten-1-ol, 3-Methylbut-3-en-1-ol, 3-Methylbut-2-en-1-ol, 1-Penten-4-ol, 3-Hexen-1-ol, 3-Methylpent-3-en-1-ol, 3-Ethylbut-3-en-1-ol, 2-Methylhex-1-en-5-ol, 2-Methylhex-1-en-4-ol, 2-Phenylbut-1-en-4-ol, 4-Methylpent-3-en-1-ol und 2-Cyclohexylbut-1-en-4-ol genannt. Besonders bevorzugter Alkohol der Formel IV ist 3-Methylbut-3-en-1-ol, auch unter dem Trivialnamen Isoprenol bekannt. Auch 3-Methylbut-2-en-1-ol, dem Fachmann unter dem Trivialnamen Prenol bekannt, kann bevorzugt eingesetzt werden.

Die Alkohole der Formel IV sind bekannt und hinsichtlich ihrer Herstellung in der Literatur beschrieben.

Nach dem erfindungsgemäßen Verfahren können Mischungen verschiedener Alkohole der Formel IV oder die entsprechenden reinen Alkohole als Ausgangsverbindungen eingesetzt werden.

Als vorteilhaft hat sich die Verwendung von Gemischen aus 3-Methylbut-3-en-1-ol (Isoprenol) und 3-Methylbut-2-en-1-ol (Prenol) herausgestellt. Prinzipiell kann jedes beliebige Mischungsverhältnis dieser Alkohole angewandt werden, als besonders gut mit Hinblick auf maximale Prenalselektivitäten hat sich ein Verhältnis von 1:1 bis 1:2 (Isoprenol/Prenol, mol/mol) erwiesen. Bei Verwendung von reinem Isoprenol, Prenol oder anderen Mischungsverhältnissen derselben miteinander stellt sich während der Reaktion in manchen Fällen zunächst das erwähnte Verhältnis von etwa 1:2 (Isoprenol/Prenol, mol/mol) unter oxidativen Bedingungen ein. Als bevorzugtes Substrat für die dann einsetzende Oxidationsreaktion dient nicht Isoprenol, wie nach dem Stand der Technik zu erwarten wäre, sondern Prenol selbst, das u. U. durch Isomerisierung gebildet wird.

Erfindungsgemäß wird die oxidative Dehydrierung bei Temperaturen im Bereich von 50 bis 240°C, vorzugsweise im Bereich von 100 bis 200°C und besonders bevorzugt im Bereich von 100 bis 150°C durchgeführt. In der Literatur werden oxidative Dehydrierungen entsprechender Alkohole in der Regel bei Temperaturen von oberhalb 300°C durchgeführt (vgl. EP-A 244 632 und EP-A 881 206), was angesichts dieser hohen Reaktionstemperaturen die Gefahr von Nebenreaktionen und Zersetzung der Edukte wie auch der gewünschten Reaktionsprodukte in sich birgt. Die niedrigeren Temperaturen nach dem erfindungsgemäßen Verfahren stellen demgegenüber einen erheblichen und technisch relevanten Vorteil dar.

Erfindungsgemäß wird die Umsetzung in einer sauerstoffhaltigen Atmosphäre, d. h. z.B. mit einem Sauerstoff enthaltenden Gas als oxidierendem Agens durchgeführt. Als entsprechendes oxidierendes Agens lassen sich dabei Gase oder Gasmischungen mit einem Sauerstoffgehalt im Bereich von 7-30 Vol.-%, vorzugsweise von 9-18 Vol.-% einsetzen. Auch Luft kann als leicht zugängliches Oxidationsmedium eingesetzt werden. Alternativ eignet sich auch Wasserstoffperoxid als oxidierendes Agens.

Gemäß der Erfindung wird als geträgerter, d.h. auf einem Träger aufgebrachter Katalysator ein Gold enthaltender geträgerter Katalysator oder ein entsprechender Katalysator der neben Gold noch ein weiteres Edelmetall ausgewählt aus Cu, Ag, Pd, Pt, Rh, Ru, W oder Os enthalten kann, eingesetzt. Durch die Verwendung von Gold oder Mischungen aus Gold und anderen Edelmetallen lässt sich die oxidative Dehydrierung bei deutlich niedrigeren Temperaturen als bislang im Stand der Technik beschrieben, durchführen. Insbesondere bei der bevorzugten Herstellung von 3-Methylbut-2-en-1-al lassen sich Reaktionstemperaturen von unter 150°C realisieren, was für die Produktqualität vorteilhaft ist und unerwünschte Nebenreaktionen zurückdrängt.

Das molare Verhältnis von Gold zu den anderen Edelmetallen unterliegt dabei keiner besonderen Beschränkung und kann frei gewählt werden.

Bei der Verwendung von 3-Methylbut-3-en-1-ol (Isoprenol) als Alkohol der Formel IV hat sich die Verwendung von geträgerten Katalysatoren, die neben Gold ein Edelmetall ausgewählt aus Pd, Pt oder Ru enthalten, bewährt. Diese geträgerten Katalysatoren katalysieren die Isomerisierung von Isoprenol zu Prenol (3-Methylbut-2-en-1-ol), welches dann zu 3-Methylbut-2-en-1-al umgesetzt werden kann, ohne dass es eines nachgeschalteten Isomerisierungsschritttes (von 3-Methylbut-3-en-1-al zu 3-Methylbut-2-en-1-al) bedarf.

Der Edelmetallgehalt der geträgerten Katalysatoren, die im erfindungsgemäßen Verfahren eingesetzt werden, unterliegt an sich keiner besonderen Beschränkung und kann im Bereich von 0,1 bis 10 Gew.-%, vorzugsweise im Bereich von 0,4 bis 5 Gew.-% und besonders bevorzugt im Bereich von 0,6 bis 3 Gew.-% liegen.

Als Trägermaterial für den Katalysator werden im Rahmen der vorliegenden Erfindung Aluminiumoxid, ein Alumosilikat oder ein Hydrotalcit oder deren Mischungen eingesetzt. Insbesondere Aluminiumoxide und Hydrotalcite haben sich in einigen Fällen als vorteilhaft erwiesen.

Verfahren zur Herstellung geeigneter Trägermaterialien sind dem Fachmann an sich ebenfalls bekannt und in der Literatur beschrieben. Nur beispielhaft sei hier für die Herstellung von Hydrotalciten als Trägermaterialien auf Cavani et al., Catal. Today, 1991, 11, Seiten 173ff. verwiesen.

Unter Hydrotalcit wird dabei allgemein ein Schichtmaterial mit der chemischen Formel [M(II)_{1-□}M(III)ₓ(OH)₂]^{x+} [A_{n/x}]ⁿ- m H₂O verstanden. Dabei steht M(II) für ein zweiwertiges Metall, M(III) für ein dreiwertiges Metall, A ist ein im Gitter eingelagertes Anion, m steht für die Zahl der eingelagerten Wassermoleküle und x steht für das molare Verhältnis M(II)/[M(II) + M(III)]. Üblicherweise liegt x im Bereich von 0,2 bis 0,33, was molaren Verhältnissen von M(II) zu M(III) im Bereich von 2 bis 4 entspricht. Als zweiwertige Metalle seien hier beispielsweise Mg, Fe, Ni, Co, Zn und Mn, als dreiwertige Metalle Al, Ga, In, Co und Mn genannt. Die Möglichkeit der gleichzeitigen Anwesenheit mehrerer zweiwertiger oder dreiwertiger Metalle in unterschiedlichen molaren Verhältnissen erhöht die Strukturvielfalt der geeigneten Hydrotalcite.

Als Minerale der Hydrotalcit-Gruppe seien hier nur beispielhaft Manasseit, Pyroaurit, Sjögrenit, Stichtit, Barbertonit, Desautelsit, Meixnerit oder Takovit erwähnt, die in der Literatur beschrieben und in ihrer Zusammensetzung dem Fachmann bekannt sind. Ein bevorzugtes Hydrotalcit hat die Zusammensetzung Mg₆Al₂(CO₃) (OH)₁₆ □ 4 H₂O.

Hydrotalcit wird aufgrund seiner Fähigkeit, durch graduelle Abgabe von Aluminiumhydroxid Säuren zu binden, vielfältig in der Industrie und als Arzneimittel eingesetzt.

Bevorzugte Varianten von Aluminiumoxid sind basische Aluminiumoxide oder Alumosilikate, wie sie ebenfalls dem Fachmann bekannt und in der Literatur beschrieben sind.

Die Herstellung der erfindungsgemäß verwendeten geträgerten Katalysatoren kann nach an sich dem Fachmann bekannten und in der Literatur beschriebenen Verfahren erfolgen. So ist beispielsweise in der EP-A 172 565 oder der EP-A 357 292 ein Verfahren zur Herstellung von geträgerten Silberkatalysatoren beschrieben, das entsprechend angepasst auch für die Herstellung der Katalysatoren der vorliegenden Erfindung verwendet werden kann. Weiter erwähnt sei hier die Herstellung der im erfindungsgemäßen Verfahren eingesetzten Trägerkatalysatoren über das sog. Flammenspritzverfahren (Beschreibung der Technologie z.B. in Army Engineering Manual EM 1110-2-3401) oder aber eine Herstellung in Anlehnung an das in Angew. Chem. Int. Ed. 2007, 47, 138-141 beschriebene Verfahren genannt.

Wegen der einfacheren Herstellungsweise werden Katalysatoren nach dem in der letzten Literaturstelle beschriebenen Verfahren bevorzugt.

Bei der erfindungsgemäß bevorzugten Herstellung von 3-Methylbut-2-en-1-al wird nach der oxidativen Dehydrierung das entstehende Reaktionsgemisch aus 3-Methyl-but-2-en-1-al und 3-Methylbut-3-en-1-al in an sich bekannter Weise einer Isomerisierung unter basischen Bedingungen unterworfen, um das gewünschte Endprodukt 3-Methylbut-2-en-1-al zu erhalten. Entsprechende Verfahren hierfür sind dem Fachmann bekannt und in der Literatur beschrieben.

Überraschenderweise hat es sich herausgestellt, dass dieser vorstehend beschriebene separate Isomerisierungsschritt bei der Verwendung von basischen Trägermaterialien als Träger für den Katalysator im erfindungsgemäßen Verfahren entfallen kann, da in diesem Fall das gewünschte Endprodukt 3-Methylbut-2-en-1-al ohne separaten Isomerisierungsschritt entsteht. Demgemäß werden derartige Katalysatoren mit basischen Trägermaterialien im erfindungsgemäßen Verfahren bevorzugt eingesetzt.

Die nachfolgenden Beispiele stellen bevorzugte Ausführungsformen des erfindungsgemäßen Verfahrens dar und dienen zur weiteren Erläuterung der Erfindung.

### Beispiel 1:

### Herstellung eines für das erfindungsgemäße Verfahren geeigneten geträgerten Katalysators auf Hydrotalcit als Träger

In einem 2000 mL Rundkolben mit Überkopfrührer und Rückflusskühler wurde zu einer Lösung von NaOH (18,6 g, 465 mmol) und Na₂CO₃ (14,2 g, 134 mmol) in Wasser (130 mL) eine Lösung von MgCl₂ x 6 H₂O (31,2 g, 154 mmol) und AlCl₃ x 6 H₂O (12,3 g, 51 mmol) in Wasser (1000 mL) bei Raumtemperatur langsam zugegeben. Dann wurde die Reaktionsmischung bei 65°C über Nacht gerührt. Der Rückstand wurde filtriert, mit Wasser bis zur Neutralität gewaschen und bei 110 °C getrocknet.

In einem 500 ml Rührkolben mit Überkopfrührer und Rückflusskühler wurde zu einer Lösung von AuCl₃ (140 mg) in Wasser (300 mL) Hydrotalcit (3,98 g aus obiger Reaktion) bei Raumtemperatur langsam hinzugefügt und für 12 h bei 60°C gerührt. Der Rückstand wurde filtriert, mit Wasser gewaschen, bis der pH neutral war und bei Raumtemperatur für 12 h getrocknet. Der Goldgehalt des so gewonnenen Katalysators betrug 1,50 Gew.-%.

### Beispiel 2:

### Herstellung eines geträgerten Katalysators auf Aluminiumoxid als Träger

In einer geschlossenen Rührapparatur wurde Goldsäure (3,41 g) in Wasser (1000 mL) gelöst und 15-20 min. gerührt. Die Lösung wurde auf 70°C erhitzt und der pH unter heftigem Rühren mit einer 0.5_{N} NaOH Lösung (86,5 g, 43,3 mmol) eingestellt. Dann wurde Al₂O₃ (50 g) zugegeben und 1 h bei 70 °C gerührt. Die Reaktionslösung wurde abgekühlt, filtriert und gewaschen. Das Wasser wurde im Vakuum entfernt, der Katalysator getrocknet und kalziniert. Der Goldgehalt des so gewonnenen Katalysators beträgt 0,98 Gew.-%.

### Beispiel 3:

### Oxidation von Isoprenol zu Prenal nach dem erfindungsgemäßen Verfahren

In einem Rundkolben mit Rückflusskühler wurde zu einer Lösung von Isoprenol (550 mg, 6,4 mmol) in *p*-Xylol (25 mL) der Katalysator (510 mg, 1,5 Gew.-% Au/Hydrotalcit) gegeben, die Reaktion wurde bei 130°C mit Luft überlagert und für 6 h gerührt. Filtration des Katalysators ergab 16,8 g einer gelblichen Flüssigkeit folgender Zusammensetzung: 1,63 Gew.-% Prenal (3-Methylbut-2-en-1-al, 3,27 mmol), 1,42 Gew.-% Isoprenol (2,79 mmol), entsprechend 56,4% Umsatz, 88,1% Selektivität und 51,1% Ausbeute.

### Beispiel 4:

### Herstellung von Prenal mit einem Trägerkatalysator auf Aluminiumoxid-Basis

Unter oben beschriebenen Bedingungen wurden Au/Al₂O₃ (0.98 Gew.-%, 5.12 g) und Isoprenol (17,2 g, 198 mmol) in *p*-Xylol (80 mL) bei 110°C umgesetzt. Filtration des Katalysators ergab 92,5 g einer gelblichen Flüssigkeit folgender Zusammensetzung: 3,29 Gew.-% Prenal (36,2 mmol), 14,3 Gew.-% Isoprenol (157,3 mmol), entsprechend 21,3% Umsatz, 85,1% Selektivität und 18,1% Ausbeute.

## Patentansprüche

1. Verfahren zur Herstellung von olefinisch ungesättigten Carbonylverbindungen der allgemeinen Formel I 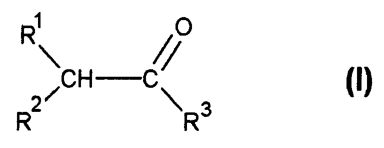 wobei R¹ Wasserstoff ist, wenn R² für einen Rest der Formel II 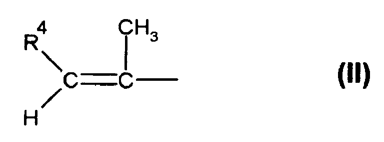 steht oder R¹ und R² zusammen einen Rest der allgemeinen Formel III bedeuten 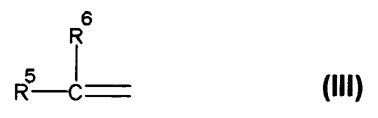 wobei R³, R⁴, R⁵ und R⁶ jeweils unabhängig voneinander für Wasserstoff, eine C₁ - C₁₈-Alkyl-, eine ggf. substituierte C₅ - C₁₈-Cycloalkyl-, oder eine ggf. substituierte C₆ - C₁₈-Arylgruppe stehen, und die Ringatome der Cycloalkyl- und Arylgruppen teilweise durch Heteroatome, ausgewählt aus N, O und S substituiert sein können,
durch Umsetzung von Alkoholen der allgemeinen Formel IV 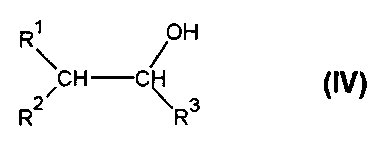 wobei R¹, R² und R³ die vorstehend genannte Bedeutung haben,
durch oxidative Dehydrierung in sauerstoffhaltiger Atmosphäre an einem goldhaltigen Katalysator, **dadurch gekennzeichnet, dass** als goldhaltiger Katalysator ein geträgerter Katalysator verwendet wird, der Gold oder eine Mischung aus Gold und Edelmetallen ausgewählt aus Cu, Ag, Pd, Pt, Rh, Ru, W oder Os enthält, die Umsetzung bei Temperaturen im Bereich von 50 bis 240°C und in Anwesenheit von Luft, Wasserstoffperoxid oder einem Gas oder Gasgemisch mit einem Sauerstoffgehalt im Bereich von 7 bis 30 Vol.-% als sauerstoffhaltige Atmosphäre durchgeführt wird, wobei
als Trägermaterial Aluminiumoxid, ein Alumosilikat oder ein Hydrotalcit oder deren Mischungen eingesetzt wird.

2. Verfahren nach Anspruch 1, **dadurch gekennzeichnet, dass** die Umsetzung bei einer Temperatur im Bereich von 80 bis 200°C durchgeführt wird.

3. Verfahren nach Anspruch 2, **dadurch gekennzeichnet, dass** die Umsetzung bei einer Temperatur im Bereich von 100 bis 150°C durchgeführt wird.

4. Verfahren nach einem der Ansprüche 1 bis 3, **dadurch gekennzeichnet, dass** als Alkohol der Formel IV 3-Methylbut-3-en-1ol eingesetzt wird.

5. Verfahren nach einem der Ansprüche 1 bis 3, **dadurch gekennzeichnet, dass** als Alkohol eine Mischung aus 3-Methylbut-3-en-1-ol und 3-Methylbut-2-en-1-ol eingesetzt wird.

6. Verfahren nach Anspruch 4, **dadurch gekennzeichnet, dass** im Reaktionsprodukt noch enthaltenes 3-Methylbut-3-en-1al zum gewünschten 3-Methylbut-2-en-1al in an sich bekannter Weise isomerisiert wird.

7. Verwendung eines geträgerten Katalysators, der Gold oder eine Mischung aus Gold mit weiteren Edelmetallen ausgewählt aus Cu, Ag, Pd, Pt, Rh, Ru, W oder Os als katalytisch aktive Spezies enthält, zur Herstellung von olefinisch ungesättigten Carbonylverbindungen der allgemeinen Formel I in Anwesenheit von Luft, Wasserstoffperoxid oder einem Gas oder Gasgemisch mit einem Sauerstoffgehalt von 7 bis 30 Vol.-% bei Temperaturen im Bereich von 50 bis 240°C, wobei als Trägermaterial Aluminiumoxid, ein Alumosilikat oder ein Hydrotalcit oder deren Mischungen eingesetzt wird.

## Claims

1. A process for preparing olefinically unsaturated carbonyl compounds of the general formula I 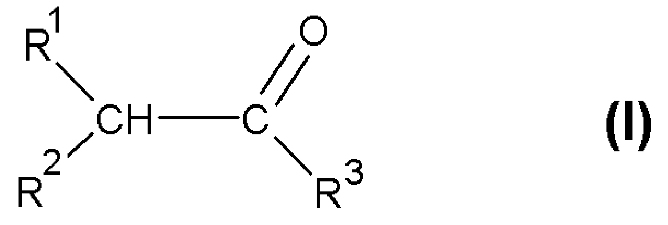 where R¹ is hydrogen when R² is a radical of the general formula II 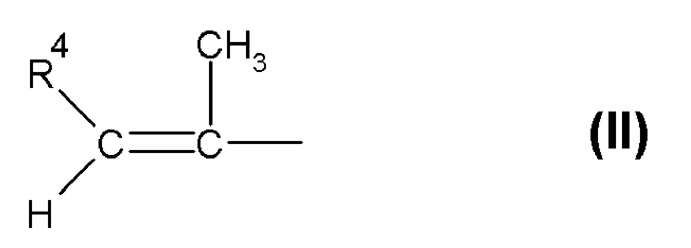 or R¹ and R² together are a radical of the general formula III 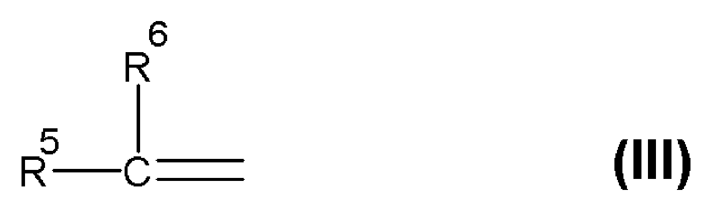 where R³, R⁴, R⁵ and R⁶ are each independently hydrogen, a C₁ - C₁₈-alkyl group, an optionally substituted C₅-C₁₈ -cycloalkyl group or an optionally substituted C₆-C₁₈-aryl group, and some of the ring atoms of the cycloalkyl and aryl groups may be replaced by heteroatoms selected from N, 0 and S,
by conversion of alcohols of the general formula IV 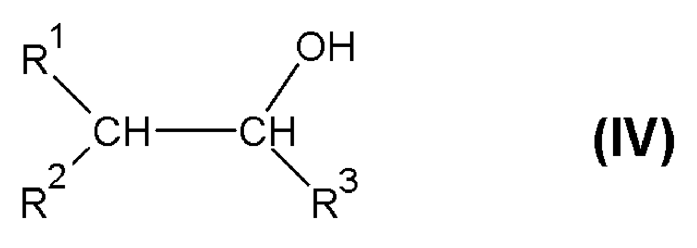 where R¹, R² and R³ are each as defined above,
by oxidative dehydrogenation in an oxygenous atmosphere over a gold catalyst, which comprises using, as the gold catalyst, a supported catalyst which comprises gold or a mixture of gold and noble metals selected from Cu, Ag, Pd, Pt, Rh, Ru, W and Os, and performing the conversion at temperatures in the range from 50 to 240°C and in the presence of air, hydrogen peroxide or a gas or gas mixture with an oxygen content in the range from 7 to 30% by volume as the oxygenous atmosphere, wherein the support material used is aluminum oxide, an alumosilcate or a hydrotalcite or mixtures thereof.

2. The process according to claim 1, wherein the conversion is performed at a temperature in the range from 80 to 200 °C.

3. The process according to claim 2, wherein the conversion is performed at a temperature in the range from 100 to 150 °C.

4. The process according to any of claims 1 to 3, wherein the alcohol of the formula IV used is 3-methylbut-3-en-1-ol.

5. The process according to any of claims 1 to 3, wherein the alcohol used is a mixture of 3-methylbut-3-en-1-ol and 3-methylbut-2-en-1-ol.

6. The process according to claim 4, wherein 3-methylbut-3-en-1-al still present in the reaction product is isomerized to the desired 3-methylbut-2-en-1-al in a manner known per se.

7. The use of a supported catalyst which comprises gold or a mixture of gold with further noble metals selected from Cu, Ag, Pd, Pt, Rh, Ru, W and Os as a catalytically active species for preparing olefinically unsaturated carbonyl compounds of the general formula I in the presence of air, hydrogen peroxide or a gas or gas mixture with an oxygen content of 7 to 30% by volume at temperatures in the range from 50 to 240 °C, wherein the support material used is aluminum oxide, an alumosilcate or a hydrotalcite or mixtures thereof.

## Revendications

1. Procédé pour la préparation de composés carbonyle oléfiniquement insaturés de formule générale I, 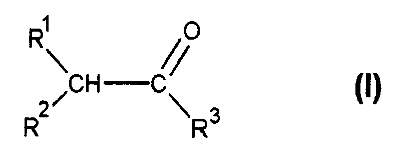 dans laquelle R¹ représente hydrogène lorsque R² représente un radical de formule II 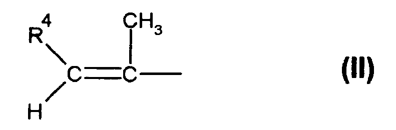 ou R¹ et R² représentent, ensemble, un radical de formule générale III 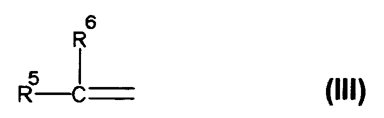 dans lesquelles R³, R⁴, R⁵ et R⁶ représentent, à chaque fois indépendamment les uns des autres, hydrogène, un groupe C₁-C₁₈-alkyle, un groupe C₅-C₁₈-cycloalkyle le cas échéant substitué ou un groupe C₆-C₁₈-aryle le cas échéant substitué, et les atomes de cycle des groupes cycloalkyle et aryle peuvent être partiellement substitués par des hétéroatomes, choisis parmi N, 0 et S, par transformation d'alcools de formule générale IV 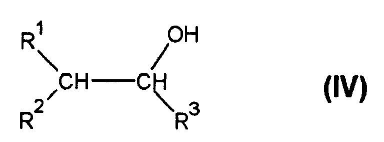 dans laquelle R¹, R² et R³ présentent la signification susmentionnée, par déshydrogénation oxydante dans une atmosphère contenant de l'oxygène sur un catalyseur contenant de l'or, **caractérisé en ce qu'**on utilise, comme catalyseur contenant de l'or, un catalyseur supporté qui contient de l'or ou un mélange d'or et de métaux nobles choisis parmi Cu, Ag, Pd, Pt, Rh, Ru, W ou Os, la transformation est réalisée à des températures dans la plage de 50 à 240°C et en présence d'air, de peroxyde d'hydrogène ou d'un gaz ou d'un mélange gazeux présentant une teneur en oxygène dans la plage de 7 à 30% en volume comme atmosphère contenant de l'oxygène ; de l'oxyde d'aluminium, un aluminosilicate ou un hydrotalcite ou leurs mélanges étant utilisés comme matériaux support.

2. Procédé selon la revendication 1, **caractérisé en ce que** la transformation est réalisée à une température dans la plage de 80 à 200°C.

3. Procédé selon la revendication 2, **caractérisé en ce que** la transformation est réalisée à une température dans la plage de 100 à 150°C.

4. Procédé selon l'une quelconque des revendications 1 à 3, **caractérisé en ce qu'**on utilise, comme alcool de formule IV, du 3-méthylbut-3-én-1-ol.

5. Procédé selon l'une quelconque des revendications 1 à 3, **caractérisé en ce qu'**on utilise, comme alcool, un mélange de 3-méthylbut-3-én-1-ol et de 3-méthylbut-2-én-1-ol.

6. Procédé selon la revendication 4, **caractérisé en ce que** le 3-méthylbut-3-én-1-al encore contenu dans le produit de réaction est isomérisé de manière connue en soi en 3-méthylbut-2-én-1-al souhaité.

7. Utilisation d'un catalyseur supporté, qui contient de l'or ou un mélange d'or et d'autres métaux nobles, choisis parmi Cu, Ag, Pd, Pt, Rh, Ru, W ou Os comme substance catalytiquement active, pour la préparation de composés carbonyle oléfiniquement insaturés de formule générale I en présence d'air, de peroxyde d'hydrogène ou d'un gaz ou d'un mélange gazeux présentant une teneur en oxygène de 7 à 30% en volume à des températures dans la plage de 50 à 240°C, de l'oxyde d'aluminium, un aluminosilicate ou un hydrotalcite ou leurs mélanges étant utilisés comme matériaux support.
